(19) Europäisches Patentamt
European Patent Office
Office européen des brevets

(11) EP 4 021 885 B1

(12) EUROPÄISCHE PATENTSCHRIFT

(45) Veröffentlichungstag und Bekanntmachung des Hinweises auf die Patenterteilung:
**12.06.2024 Patentblatt 2024/24**

(21) Anmeldenummer: **20760487.7**

(22) Anmeldetag: **27.08.2020**

(51) Internationale Patentklassifikation (IPC):
***C07C 209/36*** *(2006.01)* ***C07C 211/46*** *(2006.01)*
***B01J 23/72*** *(2006.01)*

(52) Gemeinsame Patentklassifikation (CPC):
(C-Sets verfügbar)
**C07C 209/36** (Forts.)

(86) Internationale Anmeldenummer:
**PCT/EP2020/073991**

(87) Internationale Veröffentlichungsnummer:
**WO 2021/037990 (04.03.2021 Gazette 2021/09)**

(54) **VERFAHREN ZUR HYDRIERUNG VON AROMATISCHEN NITROVERBINDUNGEN**
METHOD FOR HYDROGENATING AROMATIC NITRO COMPOUNDS
PROCÉDÉ D'HYDROGÉNATION DE COMPOSÉS NITRO AROMATIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.08.2019 EP 19194563**
**10.06.2020 EP 20179290**

(43) Veröffentlichungstag der Anmeldung:
**06.07.2022 Patentblatt 2022/27**

(73) Patentinhaber:
- **Covestro Deutschland AG**
  **51373 Leverkusen (DE)**
- **LANXESS Deutschland GmbH**
  **50569 Köln (DE)**

(72) Erfinder:
- **SANDBRINK, Lennart**
  **40764 Langenfeld (DE)**
- **PENNEMANN, Bernd**
  **51467 Bergisch Gladbach (DE)**
- **ZIRNGIEBL, Eberhard**
  **51061 Köln (DE)**
- **GERDINAND, Martina**
  **51467 Bergisch Gladbach (DE)**
- **QUELLA, Hans-Jürgen**
  **51379 Leverkusen (DE)**
- **ULLRICH, Daniel**
  **40789 Monheim am Rhein (DE)**
- **WILLIAMS, Marc**
  **51065 Köln (DE)**

(74) Vertreter: **Levpat**
**c/o Covestro AG**
**Gebäude K12**
**51365 Leverkusen (DE)**

(56) Entgegenhaltungen:
**EP-A1- 0 696 573** **EP-A1- 3 320 969**
**WO-A1-98/53910** **WO-A2-2010/130604**
**DE-A1- 3 933 661**

EP 4 021 885 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C07C 209/36, C07C 211/46**

## Beschreibung

Die vorliegende Erfindung betrifft ein Verfahren zur Herstellung eines aromatischen Amins durch Hydrierung einer aromatischen Nitroverbindung, umfassend die Schritte (I) Bereitstellen eines Kupfertetramminsalz-basierten Tränkkatalysators, insbesondere eines nach der *incipient wetness*-Methode erhältlichen Tränkkatalysators, umfassend ein Metall oder Metalloxid auf einem Träger als Hydrierkatalysator, wobei mindestens metallisches oder oxidisches Kupfer (insbesondere CuO) zugegen ist und der auf alle vorhandenen Metalle bezogene Stoffmengenanteil von Cu im Bereich von 0,75 bis 1 liegt, und wobei der Träger Siliciumdioxid-Formkörper oder Siliciumcarbid-Formkörper umfasst; (**II**) optional, Aktivieren des Hydrierkatalysators durch Behandeln mit Wasserstoff in Abwesenheit der aromatischen Nitroverbindung; sowie (**III**) Umsetzen der aromatischen Nitroverbindung mit Wasserstoff in Gegenwart des, gegebenenfalls aktivierten, Hydrierkatalysators unter Erhalt des aromatischen Amins.

Die Hydrierung von Nitroaromaten zu den korrespondierenden aromatischen Aminen mit Wasserstoff ist schon seit langem bekannt und industriell von großer Bedeutung. Als repräsentatives Beispiel sei die Hydrierung von Nitrobenzol zu Anilin genannt. Der Hauptteil des weltweit produzierten Anilins wird für die Herstellung der Di- und Polyamine der Diphenylmethanreihe (MDA), die ihrerseits Intermediate für die Herstellung der bedeutenden Di- und Polyisocyanate der Diphenylmethanreihe (MDI) sind, eingesetzt.

Die Hydrierung kann in der Flüssig- oder Gasphase, unter isothermen oder adiabatischen Bedingungen betrieben werden. Auch eine Kombination von isothermer und adiabatischer Reaktionsführung ist bekannt. Eine Reihe von Katalysatoren wurden in der Literatur für diesen Zweck beschrieben. Insbesondere sind hier Palladium- und Kupfer-basierte Katalysatorsysteme zu nennen.

So ist beispielsweise die Verwendung von Palladium-basierten Katalysatoren auf keramischen Trägern bekannt. In der deutschen Patentanmeldung DE 28 49 002 A1 wird ein Verfahren zur Reduktion von Nitroverbindungen in Gegenwart von Palladium-haltigen Drei-Komponenten-Trägerkatalysatoren in gekühlten Rohrreaktoren beschrieben. Der Katalysator enthält in bevorzugten Ausführungsformen 1 bis 20 g Palladium, 1 bis 20 g Vanadium und 1 bis 20 g Blei pro Liter $\alpha$-$Al_2O_3$. Ähnliche Katalysatoren, allerdings zusätzlich dotiert mit Mo, Re oder W, wurden auch in DE 197 15 746 A1 beschrieben. EP 1 882 681 A1 offenbart, dass es vorteilhaft ist, solche Drei-Komponenten-Trägerkatalysatoren zusätzlich mit einer Schwefel- oder Phosphor-haltigen, bevorzugt Phosphor-haltigen, Verbindung (wie beispielsweise den Sauerstoffsäuren des Phosphors oder deren Alkalisalzen wie insbesondere Natriumdihydrogenphosphat, Natrium- oder Kaliumphosphat oder Natriumhypophosphit) zu dotieren. In der internationalen Veröffentlichung WO 2013/030221 A1 werden die vorteilhaften Auswirkungen einer Kalium-Dotierung des Katalysators auf den Phenolgehalt des gebildeten Anilins beschrieben.

Die Verwendung von Kupfer-basierten Katalysatoren für die Hydrierung von insbesondere Nitrobenzol ist schon lange bekannt (siehe US 1,207,802 und US 3,136,818). Als Träger des katalytisch aktiven Materials wurde das natürliche Gestein Bims eingesetzt, was als Hauptkomponenten Silicate sowie Natrium beinhaltet.

Die Verwendung von Kupfer-Katalysatoren auf Siliciumdioxid-Trägern für die Hydrierung von Nitrobenzol zu Anilin ist ebenfalls lange bekannt (z. B. GB 823,026 oder US 2,891,094 aus den 1950er Jahren). Beide Patente beschreiben die Verwendung von Kupfer-Ammin-Komplexen als Katalysator-Vorläuferverbindungen. Zur Herstellung der Katalysatoren wird durch Ansäuern einer Natriumsilicat-Lösung ein Hydrogel ausgefällt und dieses, nach Filtration und Wäsche, mit dem Kupfer-Ammin-Komplex versetzt. Das so behandelte Hydrogel wird abfiltriert, gewaschen, getrocknet und in reduzierender Atmosphäre kalziniert. Die Behandlung des Hydrogels mit dem Kupfer-Ammin-Komplex wird zwar als Imprägnierung *bezeichnet,* jedoch entspricht der beschriebene Vorgang aufgrund der feinteiligen Natur des Trägers (der als frisch gefälltes Hydrogel vorliegt und daher gar nicht über Poren, die Kupferpartikel aufnehmen können, verfügt) eher einem einfachen *Ablagern* von Kupferpartikeln auf dem Hydrogel.

Eine bewährte und vielfach angewandte Methode zur Herstellung von Hydrierkatalysatoren ist die *Tränkung* mit Metallsalzlösungen, bei welcher der eingesetzte Träger über Poren verfügt, welche die Metallsalzlösungen aufnehmen. Zu diesem Zweck wird der Träger entweder maximal bis zur Sättigung seiner Wasseraufnahmekapazität mit der Metallsalzlösung befeuchtet (sog. *"incipient wetness"*-Methode) oder in überstehender Lösung behandelt. Tränkverfahren sind beispielsweise in den nachfolgend diskutierten Patentanmeldungen WO 2010/130604 A2, EP 0 696 573 A1, DE 2 311 114, WO 95/32171 A1 und WO 2009/027135 A1 beschrieben:

Die internationale Patentanmeldung WO 2010/130604 A2 beschreibt ein Verfahren zur Herstellung von aromatischen Aminen, insbesondere Anilin, unter Verwendung von Kupfer-haltigen Katalysatoren auf $SiO_2$-Trägern. Das Verfahren ist insbesondere dadurch gekennzeichnet, dass das $SiO_2$ durch Nassmahlung und anschließende Sprühtrockung hergestellt wurde. Der Nassmahlprozess liefert Siliciumdioxidpartikel mit einem Durchmesser in der Größenordnung von Mikrometern, insbesondere im Bereich von 1 bis 35 $\mu$m. Derart kleine Katalysatordurchmesser sind für das beschriebene Verfahren auch erforderlich, denn die so hergestellten Katalysatoren sollen als Wirbelschichtkatalysatoren eingesetzt werden, was mit makroskopisch fassbaren Formkörpern mit Größen im Milimeterbereich gar nicht praktikabel wäre. Zur Aufbringung der katalytisch aktiven Metalle wird die Tränkung aus überstehender Lösung beschrieben, beispielsweise unter Verwendung ammoniakalischer Carbonatlösungen.

EP 0 696 573 A1 beschreibt ein Verfahren, in dem aromatische Amine durch Hydrierung der zugehörigen Nitroaromaten in der Gasphase an ortsfesten Katalysatoren hergestellt werden. Die Katalysatoren enthalten hydrieraktive Metalle auf Trägern, die durch Tränkung hergestellt werden können. Als Hydrierkatalysator wird insbesondere ein Palladium auf $\alpha$-$A1_2O_3$ enthaltender Katalysator eingesetzt, der 1 bis 100 g Pd pro Liter $\alpha$-$A1_2O_3$ enthält, das bevorzugt schalenförmig niedergeschlagen ist, wobei der Katalysator zusätzlich Vanadium und Blei enthalten kann. Katalysatoren, die auf Amminkomplexen basieren, werden nicht beschrieben.

Die deutsche Offenlegungsschrift DE 2 311 114 befasst sich mit der Verbesserung von zur Hydrierung von Ketonen, Carbonsäureestern und Nitroverbindungen eingesetzten Kupferchromit-Katalysatoren. Zu diesem Zweck wird ein Verfahren zur Herstellung eines auf Trägern aufgebrachten Kupferchromit-Katalysators vorgeschlagen, das insbesondere dadurch gekennzeichnet ist, dass basisches Ammonium-Kupfer(II)-chromat in den Poren eines anorganischen oxidischen Trägermaterials durch Umsetzung von hier miteinander reagierenden Präcursoren des basischen Ammonium-Kupfer(II)-chromates gebildet und das Trägermaterial dann zur Umwandlung des basischen Ammonium-Kupfer(II)-chromates in Kupferchromit etwa 0,1 bis 20 Stunden auf eine Temperatur von etwa 250 bis 500 °C erhitzt wird. Gemäß dieser Schrift wird Kupferchromit oft als "$xCuO,Cr_2O_3$" dargestellt. Für den Fachmann ist unmittelbar ersichtlich, dass dies lediglich eine Beschreibung der stöchiometrischen Verhältnisse ist und noch keinen Aufschluss über die tatsächliche Struktur des Katalysators gibt. Katalysatoren mit einem Stoffmengenanteil an Cu von 75 mol-% oder mehr werden nicht offenbart.

Die internationale Patentanmeldung WO 95/32171 A1 befasst sich mit der Herstellung von Alkoholen durch die katalytische Hydrierung der entsprechenden Carbonylverbindungen bei erhöhter Temperatur und bei erhöhtem Druck in flüssiger Phase. Hierzu werden Kupfer-Katalysatoren beschrieben, die durch Tränkung von $SiO_2$-haltigen Trägermaterialien mit verschiedenen thermisch "leicht" (d. h. unterhalb von 350 °C) zersetzbaren Kupfer-Salzen wie Kupfernitrat, Kupfercarbonat, Kupferformiat, Kupferoxalat und deren gut wasserlösliche, am(m)inische Komplexe erhältlich sind.

Die internationale Patentanmeldung WO 2009/027135 A1 befasst sich ebenfalls mit der Herstellung von Alkoholen durch Hydrierung von Carbonylverbindungen. Der eingesetzte Hydrierkatalysator besteht aus einem Trägermaterial und zumindest einem hydrieraktiven Metall, wobei das Trägermaterial auf Titandioxid, Zirkondioxid, Aluminiumoxid, Siliziumoxid oder deren Mischoxiden basiert und das hydrieraktive Metall mindestens ein Element der Gruppe Kupfer, Kobalt, Nickel, Chrom ist, und wobei das Trägermaterial ferner das Element Barium enthält. Als ein Beispiel ist die Herstellung eines Kupfer-haltigen Tränkkatalysators auf Aluminiumoxid) mit einer ca. 14%igen Kupfertetrammincarbonat-Lösung beschrieben.

Von Tränkkatalysatoren völlig verschieden sind *Katalysatorlegierungen,* zum Beispiel die bekannten Raney-Katalysatoren. WO 98/53910 A1 offenbart einen geformten, aktivierten Metall-Festbettkatalysator mit einem Porenvolumen von 0,05 bis 1 ml/g und einer äußeren, aktivierten Schale, bestehend aus einer versinterten, feinteiligen Katalysatorlegierung sowie gegebenenfalls Promotoren, wobei die Katalysatorlegierung aus der Herstellung der Legierung resultierende metallurgische Phasendomänen aufweist, deren volumenmäßig größte Phase eine spezifische Grenzflächendichte von mehr als 0,5 $gm^{-1}$ besitzt.

Die deutsche Patentanmeldung DE 39 33 661 A1 befasst sich mit einem Katalysator zur Hydrierung von Acetophenon zu Methylbenzylalkohol. Der Katalysator wird durch Imprägnierung, worunter insbesondere *Besprühung* verstanden wird, eines Siliciumdioxid-Trägers mit einer Lösung von Kupfertetramincarbonat und einer Lösung von Ammoniumchromat oder mit deren Gemisch und anschließende Trocknung hergestellt.

Die deutsche Patentanmeldung DE 10 2010 029 924 A1 befasst sich mit der Regenerierung von Kupfer-, Chrom- und/oder Nickel-haltigen Hydrierkatalysatoren, wie sie in der Herstellung höherer Alkohole, insbesondere solcher mit 8 bis 13 Kohlenstoffatomen, durch katalytische Hydroformylierung (auch als Oxoreaktion bezeichnet) der um ein Kohlenstoffatom ärmeren Olefine und anschließende Hydrierung der gebildeten Aldehyde eingesetzt werden.

Das britische Patent GB 825,602 befasst sich mit der Dehydrierung von Alkoholen zu Aldehyden und Ketonen, wobei ein reduziertes und geringe Mengen nicht reduziertes Kupferoxid sowie "Alkalimetalleoxide" enthaltender Katalysator eingesetzt wird. Der Katalysator wird durch Erhitzen eines Kupfertetrammin-Komplexes gefolgt von Erhitzen unter Wasserstoff hergestellt.

Die europäische Patentanmeldung EP 3 320 969 A1 befasst sich mit Chrom- und Nickel-freien Katalysatoren zur heterogenen Hydrierung von Oxo-Aldehyden. Die Katalysatoren enthalten lediglich Kupfer; dabei ist es jedoch erforderlich, dass als Trägermaterial Siliciumdioxid eingesetzt wird und dass der Gehalt an Cu und $SiO_2$ im aktiven Katalysator in sehr engen Grenzen genau eingestellt wird.

Neben der Verwendung als Katalysatoren für die verschiedensten Reaktionen finden Kupferverbindungen auch in vielen anderen Gebieten Anwendung, so zum Beispiel als Fungizid (siehe etwa US 3,900,504**).**

Mit der Untersuchung von Kupferkatalysatoren für verschiedene Anwendungszwecke befassen sich auch verschiedene Artikel aus der Nichtpatentliteratur. Beispielhaft seien Chem. Lett. 1980, 1197 - 1200, Appl. Catal. 1982, 3, 381 - 388, Appl. Catal. 1987, 31, 309 - 321 und Procedia Engineering 2013, 51, 467 - 472 genannt.

Neben der Verwendung von Palladium- oder Kupfer-basierten Katalysatoren ist auch der Einsatz von Katalysatoren bekannt, die beide Metalle enthalten. Ein Beispiel hierfür ist in dem britischen Patent GB 961,394 beschrieben. Dort werden Katalysatoren für die Abgasreinigung von Kraftfahrzeugen beschrieben, die 0,5 bis 25 % Kupfer und 0,01 bis 3 % Palladium enthalten.

Die für die Hydrierung von Nitroaromaten, insbesondere von Nitrobenzol, beschriebenen Katalysatoren des Standes der Technik sind zwar grundsätzlich für diesen Zweck geeignet, jedoch gibt es immer noch Verbesserungspotenzial hinsichtlich Selektivität und Langzeitstabilität. Hierbei lag das Augenmerk auf den im Vergleich zu den bekannten Palladium-basierten Katalysatoren preiswerteren Kupfer-basierten Katalysatoren.

Dem vorstehend Gesagten Rechnung tragend hat die vorliegende Erfindung ein Verfahren zur Herstellung eines aromatischen Amins, insbesondere Anilin, durch Hydrierung einer aromatischen Nitroverbindung, insbesondere Nitrobenzol, zum Gegenstand, das die folgenden Schritte umfasst:

(I) Bereitstellen eines Kupfertetramminsalz-basierten Tränkkatalysators, insbesondere eines nach der *incipient wetness*-Methode erhältlichen (bevorzugt: *hergestellten*) Tränkkatalysators, umfassend ein Metall oder Metalloxid auf einem Träger als Hydrierkatalysator, wobei mindestens metallisches oder oxidisches Kupfer (insbesondere CuO) zugegen ist und der auf alle vorhandenen Metalle bezogene Stoffmengenanteil von Cu im Bereich von 0,75 bis 1, bevorzugt 0,90 bis 1 liegt, und wobei der Träger Siliciumdioxid-Formkörper oder Siliciumcarbid-Formkörper umfasst;

(II) optional (und bevorzugt), Aktivieren des Hydrierkatalysators durch Behandeln mit Wasserstoff in Abwesenheit der aromatischen Nitroverbindung;

(III) Umsetzen der aromatischen Nitroverbindung mit Wasserstoff in Gegenwart des, gegebenenfalls aktivierten, Hydrierkatalysators unter Erhalt des aromatischen Amins.

In der Terminologie der vorliegenden Erfindung wird unter einem *Kupfertetramminsalz-basierten Tränkkatalysator umfassend ein Metall oder Metalloxid auf einem Träger* ein Katalysator verstanden, der durch Tränkung eines Trägers mit einer wässrigen, insbesondere ammoniakalischen, Lösung eines Kupfertetramminsalzes (also ein Salz enthaltend den Tetramminkomplex von $Cu^{II}$, $[Cu^{II}(NH_3)_4]^{2+}$, als Kation), gefolgt von Trocknung und Kalzinierung (bevorzugt in Sauerstoff-haltiger Atmosphäre) erhalten wurde. Die Tränkung des Trägers erfolgt durch dessen Vermischen mit der wässrigen, insbesondere ammoniakalischen, Lösung eines Kupfertetramminsalzes (durch Einbringen des Trägers in die oder durch Übergießen des Trägers mit der Kupfertetramminsalzlösung). Dabei werden die Art des Trägers und die Menge der wässrigen, insbesondere ammoniakalischen, Lösung des Kupfertetramminsalzes so aufeinander abgestimmt, dass

- entweder (= Tränkung in überstehender Lösung) *mehr* Kupfertetramminsalzlösung vorhanden ist, als die Poren des Trägers (siehe hierzu auch den Abschnitt weiter unten zu *Formkörpern*) aufnehmen können,
- oder (= *incipient wetness*-Methode - bevorzugte Methode) maximal gerade so viel (bevorzugt etwas weniger, insbesondere 2 % bis 5 % weniger) Kupfertetramminsalzlösung vorhanden ist, wie die Poren des Trägers aufnehmen können.

Eine solche Tränkung des Trägers kann auch als *Imprägnierung* bezeichnet werden, wobei zu beachten ist, dass in der Terminologie der vorliegenden Erfindung der Begriff *Imprägnierung* auf die vorbeschriebene Tränkung beschränkt ist und **nicht** etwa, wie es in der Fachliteratur verschiedentlich der Fall ist, als Sammelbegriff für nahezu jede Art der Aufbringung hydrieraktiver Substanzen auf einem Träger dient.

Der Tränkkatalysator ist daher insbesondere ein solcher, der nach der erwähnten *incipient wetness-Methode* erhältlich ist (und bevorzugt tatsächlich nach dieser Methode hergestellt wurde). Dies bedeutet mit anderen Worten, dass im Herstellverfahren des Tränkkatalysators der Träger bevorzugt so mit der wässrigen, insbesondere ammoniakalischen, Lösung eines Kupfertetramminsalzes getränkt wird, dass die *mittels Sättigung mit Wasser bestimmte maximale Saugfähigkeit des Trägers* nicht überschritten wird und bevorzugt nicht mehr als 5 % unterschritten sowie weiterhin bevorzugt um mindestens 2 % unterschritten wird. Möglichkeiten zur Bestimmung der *mittels Sättigung mit Wasser bestimmten maximalen Saugfähigkeit des Trägers* sind in der Fachwelt bekannt. Maßgeblich für die Zwecke der vorliegenden Erfindung ist die im Abschnitt *"Bestimmung der maximalen Saugfähigkeit des Trägers"* zu Beginn des Beispielteils beschriebene Methode.

Der *Träger* umfasst erfindungsgemäß *Siliciumdioxid- oder Siliciumcarbid-Formkörper,* wobei in diesem Zusammenhang unter einem *Formkörper* zu verstehen ist, dass der Träger in Form diskreter (d. h. makroskopisch fassbarer) Partikel mit mittleren Durchmessern insbesondere im Bereich von 1,0 mm bis 15 mm, bevorzugt im Bereich von 4,0 mm bis 10 mm, vorliegt. Als Beispiele seien insbesondere *zylindrische Formkörper* und *kugelförmige Formkörper* genannt, wobei bei *zylindrischen Formkörpern* der Durchmesser der Grundfläche als Durchmesser in diesem Sinne gilt und die Länge der zylindrischen Formkörper stets größer als der Durchmesser ist. Im Falle von zylindrischen Formkörpern

können die einzelnen Zylinder auch zu mehrere Zylinder umfassenden Aggregaten zusammengefügt sein, insbesondere zu sog. *Trilobes* (Aggregate aus drei Zylindern, die entlang der Längsrichtung miteinander verbunden sind). *Im Falle solcher Aggregate von Zylindern* gilt als Durchmesser der Durchmesser eines gedachten, die Grundflächen der miteinander verbundenen Zylinder einhüllenden Kreises.

Solche Formkörper sind sowohl von unförmigen Strukturen (wie Staub oder Hydrogele) als auch von monolithischen Strukturen verschieden. Die Siliciumdioxid- oder Siliciumcarbid-Formkörper enthalten Poren, in welche die wässrige Lösung des Kupfertetramminsalzes eindringt. *Siliciumdioxid* ($SiO_2$), wie in der Terminologie der vorliegenden Erfindung verwendet, wird in der englischsprachigen Literatur üblicherweise als *silica* bezeichnet.

Der *der auf alle vorhandenen Metalle bezogene Stoffmengenanteil von Cu* (= *x(Cu)*) bezieht sich jeweils auf die Metalle als solche, also $x(Cu) = \frac{Stoffmenge\ Cu}{Summe\ der\ Stoffmengen\ aller\ vorhandenen\ Metalle}$. Die Massenanteile der auf dem Katalysator vorhandenen Metalle sind herstellungsseitig bekannt; daraus lässt sich der Stoffmengenanteil des Kupfers *x(Cu)* leicht errechnen. Ist außer Kupfer kein anderes Metall zugegen (was bevorzugt ist), beträgt *x(Cu)* 1.

Es folgt zunächst eine Kurzzusammenfassung verschiedener möglicher **Ausführungsformen der Erfindung,** wobei die Aufzählung an Ausführungsformen nicht als erschöpfend anzusehen ist:
In einer **ersten Ausführungsform** der Erfindung, die mit allen anderen Ausführungsformen kombiniert werden kann, wird Schritt (II) durchgeführt, wobei die Behandlung mit Wasserstoff bei Temperaturen im Bereich von 180 °C bis 240 °C erfolgt.

In einer **zweiten Ausführungsform** der Erfindung, die mit allen anderen Ausführungsformen kombiniert werden kann, wird Schritt (III)

adiabatisch bei Temperaturen im Bereich von 160 °C bis 500 °C, bevorzugt 180 °C bis 450 °C, besonders bevorzugt 200 °C bis 400 °C,
oder

isotherm bei Temperaturen im Bereich von 180 °C bis 550 °C, bevorzugt 200 °C bis 500 °C, besonders bevorzugt 220 °C bis 450 °C,

durchgeführt.

In einer **dritten Ausführungsform** der Erfindung, die mit allen anderen Ausführungsformen, insbesondere mit der zweiten, kombiniert werden kann, wird Schritt (III)

adiabatisch bei einem molaren Verhältnis von Wasserstoff zu Nitrogruppen im Bereich von 10 bis 200, bevorzugt von 20 bis 150, besonders bevorzugt von 60 bis 120,
oder

isotherm bei einem molaren Verhältnis von Wasserstoff zu Nitrogruppen im Bereich von 3 bis 100, bevorzugt von 6 bis 60, besonders bevorzugt von 10 bis 30,

durchgeführt.

In einer **vierten Ausführungsform** der Erfindung, die mit allen anderen Ausführungsformen kombiniert werden kann, liegt der Massenanteil an Kupferverbindungen, berechnet als metallisches Cu, des in (I) bereitgestellten Hydrierkatalysators, bezogen auf dessen Gesamtmasse, im Bereich von 3 % bis 35 %, bevorzugt 7 % bis 30 %, besonders bevorzugt 11 % bis 25 %.

In einer **fünften Ausführungsform** der Erfindung, die mit allen anderen Ausführungsformen kombiniert werden kann, sofern diese Siliciumdioxid-Formkörper als Träger umfassen, weist der Siliciumdioxid-Formkörper umfassende Träger eine spezifische Oberfläche im Bereich von 100 $m^2/g$ bis 350 $m^2/g$, bevorzugt im Bereich von 100 $m^2/g$ bis 300 $m^2/g$, ein Porenvolumen im Bereich von 0,3 $cm^3/g$ bis 1,5 $cm^3/g$, bevorzugt im Bereich von 0,3 $cm^3/g$ bis 1,4 $cm^3/g$ und eine Seitenbruchfestigkeit im Bereich von 40 N bis 500 N, bevorzugt im Bereich von 40 N bis 350 N auf.

In einer **sechsten Ausführungsform** der Erfindung, die mit allen anderen Ausführungsformen kombiniert werden kann, wird als Hydrierkatalysator ein Kupfertetrammincarbonat-basierter Tränkkatalysator eingesetzt.

In einer **siebten Ausführungsform** der Erfindung, die eine besondere Ausgestaltung der sechsten Ausführungsform ist, wird als Hydrierkatalysator ein Kupfertetrammincarbonat-**Ammoniumcarbonat-**basierter Tränkkatalysator oder ein Kupfertetrammincarbonat-**Ammoniumacetat-**basierter Tränkkatalysator, vorzugsweise ein Kupfertetrammincarbonat-**Ammoniumcarbonat-**basierter Tränkkatalysator, eingesetzt.

In einer **achten Ausführungsform** der Erfindung, die mit allen anderen Ausführungsformen kombiniert werden kann, umfasst Schritt (I):

(a) Lösen eines Kupfersalzes in wässrigem Ammoniak unter Erhalt einer ammoniakalkalischen Kupfersalzlösung;

(b) Tränken des Trägers mit der in (a) erhaltenen ammoniakalkalischen Kupfersalzlösung gefolgt von Trocknen des so erhaltenen getränkten (= imprägnierten) Trägers unter Erhalt einer Katalysatorvorstufe,

(c) Kalzinieren der in (c) erhaltenen Katalysatorvorstufe unter Ausbildung des Kupfertetrammin-basierten Tränkkatalysators.

In einer **neunten Ausführungsform** der Erfindung, die eine besondere Ausgestaltung der achten Ausführungsform ist, umfasst das Kupfersalz basisches Kupfercarbonat.

In einer **zehnten Ausführungsform** der Erfindung, die eine besondere Ausgestaltung der achten und neunten Ausführungsform ist, wird in Schritt (I)(a) zusätzlich zu dem Kupfersalz auch Ammoniumcarbonat in dem wässrigen Ammoniak gelöst.

In einer **elften Ausführungsform** der Erfindung, die eine besondere Ausgestaltung der achten bis zehnten Ausführungsform ist, wird das Lösen in Schritt (I)(a) bei Temperaturen im Bereich von 0,0°C bis 10,0 °C, bevorzugt 1,0 °C bis 5,0 °C, durchgeführt.

In einer **zwölften Ausführungsform** der Erfindung, die eine besondere Ausgestaltung der achten bis elften Ausführungsform ist, wird das Trocknen in Schritt (I)(b) bei Temperaturen im Bereich von 80 °C bis 150 C, bevorzugt im Bereich von 90 °C bis 130 °C, besonders bevorzugt im Bereich von 100 °C bis 120 °C, durchgeführt.

In einer **dreizehnten Ausführungsform** der Erfindung, die eine besondere Ausgestaltung der achten bis zwölften Ausführungsform ist, wird das Kalzinieren in Schritt (I)(c) bei Temperaturen im Bereich von 300 °C bis 600 °C, bevorzugt im Bereich von 350 °C bis 550 °C, besonders bevorzugt im Bereich von 400 °C bis 500 °C, durchgeführt.

In einer **vierzehnten Ausführungsform** der Erfindung, die eine besondere Ausgestaltung der achten bis dreizehnten Ausführungsform ist, weist die zum Tränken (= Imprägnieren) eingesetzte ammoniakalkalische Kupfersalzlösung einen pH-Wert (20°C) im Bereich von 7,0 bis 14, bevorzugt von 8,0 bis 12, besonders bevorzugt von 9,0 bis 11, auf.

In einer **fünfzehnten Ausführungsform** der Erfindung, die mit allen anderen Ausführungsformen kombiniert werden kann, weisen die Siliciumdioxid-Formkörper oder Siliciumcarbid-Formkörper einen mittleren Durchmesser im Bereich von 1,0 mm bis 15 mm, bevorzugt im Bereich von 4,0 mm bis 10,0 mm, auf.

In einer **sechzehnten Ausführungsform** der Erfindung, die mit allen anderen Ausführungsformen, insbesondere mit der fünfzehnten, kombiniert werden kann, sofern diese Ausführungsformen Siliciumdioxid-Formkörper als Träger umfassen, sind die Siliciumdioxid-Formkörper erhältlich durch:

(i) Ausfällen von Siliciumdioxid aus einer Silicat-Lösung und Isolieren des ausgefällten Siliciumdioxids;

(ii) Trocknen des Siliciumdioxids;

(iii) Verarbeiten des getrockneten Siliciumdioxids zu Formkörpern;

(iv) Kalzinieren der Formkörper, bevorzugt bei einer Temperatur im Bereich von 500 °C bis 1000 °C.

In einer **siebzehnten Ausführungsform** der Erfindung, die mit allen anderen Ausführungsformen kombiniert werden kann, wird eine aromatische Nitroverbindung der Formel

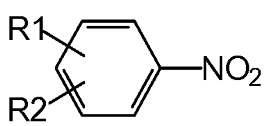

hydriert, worin R1 und R2 unabhängig voneinander Wasserstoff, Methyl oder Ethyl bedeuten, wobei R2 weiterhin auch $NO_2$ bedeuten kann.

In einer **achtzehnten Ausführungsform** der Erfindung, die mit allen anderen Ausführungsformen kombiniert werden kann, wird Nitrobenzol zu Anilin hydriert.

In einer **neunzehnten Ausführungsform** der Erfindung, die mit allen Ausführungsformen umfassend den Schritt (b) kombiniert werden kann, wird der Träger in Schritt (b) so mit der in (a) erhaltenen ammoniakalischen Kupfersalzlösung getränkt, dass die mittels Sättigung mit Wasser bestimmte maximale Saugfähigkeit des Trägers nicht überschritten wird (= *incipient wetness*-Methode).

In einer **zwanzigsten Ausführungsform** der Erfindung, die eine besondere Ausgestaltung der neunzehnten Ausführungsform ist, wird die maximale Saugfähigkeit des Trägers um nicht mehr als 5 % unterschritten.

In einer **einundzwanzigsten Ausführungsform** der Erfindung, die eine besondere Ausgestaltung der achtzehnten und neunzehnten Ausführungsform ist, wird die maximale Saugfähigkeit des Trägers um mindestens 2 % unterschritten.

In einer **zweiundzwanzigsten Ausführungsform** der Erfindung, die mit allen anderen Ausführungsformen kombiniert werden kann, wird der, gegebenenfalls aktivierte, Hydrierkatalysator in Schritt (III) in einem Katalysatorfestbett angeordnet.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

BEREITSTELLUNG DES KATALYSATORS ZUR DURCHFÜHRUNG DER HYDRIERUNG

**Schritt (I)** des erfindungsgemäßen Verfahrens umfasst das *Bereitstellen des Kupfertetramminsalz-basierten Tränkkatalysators umfassend CuO auf Siliciumdioxid-Formkörpern als Träger.* Solche Katalysatoren sind lager- und transportstabil; daher kann die Herstellung des Katalysators von der eigentlichen Hydrierung vollkommen entkoppelt werden und beispielsweise an einem anderen Standort erfolgen.

Herstellung des Trägers

Als Träger geeignete *Siliciumdioxid-Formkörper* sind nach dem Fachmann bekannten Methoden herstellbar und sind kommerziell erhältlich.

Bevorzugt werden die Siliciumdioxid-Formkörper durch Fällen von feinteiligem Siliciumdioxid aus wässrigen Silicat-Lösungen erhalten (Herstellung eines *Gels*). Das ausgefällte feinteilige Siliciumdioxid wird isoliert und zu Siliciumdioxid-Pulver getrocknet. Zur Herstellung von Siliciumdioxid-Formkörpern aus dem Siliciumdioxid-Pulver wird dieses auf dem Fachmann bekannte Weise zu Formkörpern weiterverarbeitet, bevorzugt extrudiert oder granuliert. Die Formkörper können in unterschiedlicher räumlicher Gestalt gewonnen werden; beispielsweise als Zylinder (was Aggregate von Zylindern wie die oben erwähnten *Trilobes* umfasst) oder Kugeln. Evtl. vorhandene geringfügige Abweichungen von der idealen Zylinder- oder Kugelgeometrie verlassen den Rahmen der Erfindung selbstverständlich nicht.

Als Träger geeignete *Siliciumcarbid-Formkörper* sind nach dem Fachmann bekannten Methoden herstellbar und sind kommerziell erhältlich, wobei die Reinheit der Materialien variieren kann. Insbesondere geeignet ist mesoporöses (d. h. einen Porendurchmesser im Bereich von 2 nm bis 50 nm aufweisendes) β-Siliciumcarbid. Die im Rahmen der vorliegenden Erfindung verwendete Definition von "mesoporös" folgt der entsprechenden IUPAC-Empfehlung (siehe Pure & Appl. Chem., 1994, 66, 1739 - 1758).

An die Verarbeitung zu Silciumdioxid- bzw. Siliciumcarbid-Formkörpern schließt sich bevorzugt ein Kalzinierungsschritt, vorzugsweise bei Temperaturen im Bereich von 300 °C bis 1000 °C, an. Die Kalzinierung kann in einer Sauerstoff-haltigen Atmosphäre (insbesondere in Luft), in einer Wasserstoffatmosphäre oder in einer inerten Atmosphäre (insbesondere in Stickstoff- oder Edelgasatmosphäre) durchgeführt werden. Kalzinierung in Sauerstoff-haltiger Atmosphäre ist bevorzugt.

Bevorzugt weisen Formkörper in Gestalt von Zylindern (sofern durchgeführt nach der Kalzinierung) eine mittlere Länge im Bereich von 3,0 mm bis 18 mm, bevorzugt im Bereich von 6,0 mm bis 14 mm, und einen mittleren Durchmesser im Bereich von 1,0 mm bis 15 mm, bevorzugt im Bereich von 4,0 mm bis 10 mm, auf. Dies kann durch Einstellung entsprechender Bedingungen bei der Extrusion erreicht und durch einfache Messung (z. B. mittels einer Schieblehre) überprüft werden.

Bevorzugt weisen kugelförmige Formköper (sofern durchgeführt nach der Kalzinierung) eine mittlere Partikelgröße (d. h. die mittlere Größe der Formkörper = mittlerer Durchmesser) $x_{50,3}$ im Bereich von 1,0 mm bis 15 mm, besonders bevorzugt im Bereich von 4,0 mm bis 10,0 mm auf. Dies kann durch Einstellen entsprechender Bedingungen bei der Granulation und/oder durch Siebung erreicht werden. Ob die angestrebte mittlere Partikelgröße tatsächlich vorliegt, kann durch eine *Partikelgrößenanalyse* überprüft werden. Die hierzu im Rahmen der Erfindung maßgebliche Messmethode ist die *Siebanalyse.* Dabei wird als mittlere Partikelgröße der massebezogene Wert ("$x_{50,3}$") verwendet. Zur Bestimmung der mittleren Partikelgröße wird nun so vorgegangen, dass man zunächst eine repräsentative Probe der Partikel (= Formkörper) einer Siebanalyse unterwirft und das Ergebnis massebezogen auswertet. Die Siebanalyse erfolgt dabei unter Verwendung einer Vibrationssiebmaschine (z. B. Modell AS 200 digit der Firma Retsch), in welcher die Analysensiebe mit aufsteigender Maschenweite übereinander zu einem Siebsatz angeordnet werden. Die Auswahl der Analysensiebe (Durchmesser und Maschenweite) hängt in erster Linie von der Siebgutmenge und der zu erwartenden (ggf. sind Vorversuche nötig) Partikelgrößenverteilung ab. Die Anzahl der Siebe und die Abstufungen der nominalen Öffnungsweiten sollten so ausgewählt werden, dass möglichst das gesamte Partikelspektrum der Probe in Fraktionen aufgeteilt wird. Bei der Durchführung der Siebanalyse ist sicherzustellen, dass der maximale Durchgang an Siebgut (optimaler Aussiebegütegrad) erzielt wird. Erforderlichenfalls (wenn beispielsweise neue Partikel, mit denen noch keine

Betriebserfahrung vorliegt, eingesetzt werden sollen) müssen geeignete Siebzeiten und Amplituden in Vorversuchen experimentell ermittelt werden. Ein erster Anhaltspunkt für die Amplitude ergibt sich aus der Beobachtung der Siebgutbewegung. Diese sollte weder zu schwach noch zu stark sein. Die optimale Siebzeit ist erreicht, wenn sich die Masse des Siebdurchgangs in einer Minute um weniger als 0,1 % der Aufgabemenge verändert (DIN 66165, Fassung vom April 1987). Der Fachmann ist mit den hier nur kurz umrissenen Methoden vertraut. Die Siebanalyse liefert als Ergebnis die Partikelgrößenverteilung der gemessenen Partikel. Das Ergebnis wird vorzugsweise graphisch dargestellt, indem der Massenanteil der einzelnen Fraktionen ("$p_3$") in einem Balkendiagramm und die aus den prozentualen Anteilen kumulierte Summenkurve ("$Q_3$") über den nominalen Sieböffnungsweiten (x) aufgetragen werden. Der Fachmann kann die mittlere Partikelgröße $x_{50,3}$ (d. h. 50 Massen-% der Partikel sind kleiner als der entsprechende Wert x) leicht errechnen, entweder manuell oder bevorzugt durch Computer-gestützte Auswerteprogramme.

Vorzugsweise weist ein Siliciumdioxid-Formkörper umfassender Träger (sofern durchgeführt nach der Kalzinierung) eine spezifische Oberfläche im Bereich von 100 $m^2/g$ bis 350 $m^2/g$, besonders bevorzugt im Bereich von 100 $m^2/g$ bis 300 $m^2/g$ (bestimmt nach F. M. Nelsen und F. T. Eggertsen, Analyt. Chem. 1958, 30, 1387 - 1392), ein Porenvolumen im Bereich von 0,3 $cm^3/g$ bis 1,4 $cm^3/g$, bevorzugt im Bereich von 0,3 $cm^3/g$ bis 1,4 $cm^3/g$ (bestimmt nach und S. W. Sing, Adsorption, Surface Area and Porosity, London 1982, Kap. 2 und 6) und eine Seitenbruchfestigkeit im Bereich von 40 N bis 500 N, bevorzugt im Bereich von 40 N bis 350 N (bestimmt nach DIN 50 106 in der Fassung von November 2016; diese für metallische Werkstoffe beschriebene Methode ist auch für die erfindungsrelevanten Träger geeignet) auf. Für erfindungsgemäß einsetzbare mesoporöse $\beta$-Siliciumcarbid-Formkörper umfassende Träger gelten mit Ausnahme der BET-Oberfläche vergleichbare Werte. Die BET-Oberfläche ist hier typischerweise geringer und liegt bevorzugt im Bereich von 20 $m^2/g$ bis 30 $m^2/g$.

Herstellung der Lösung des Kupfertetramminsalzes

Methoden zur Herstellung von Kupfertetramminsalzen sind prinzipiell im Stand der Technik bekannt. Im Folgenden wird eine bevorzugte Vorgehensweise geschildert.

Als Ausgangs-Kupfersalz wird vorzugsweise basisches Kupfercarbonat eingesetzt. Andere Kupfersalze wie Kupferhydroxid und Kupferacetat sind jedoch auch verwendbar. Es können auch Mischungen von Kupfersalzen verwendet werden. Das Kupfersalz wird in wässrigem Ammoniak unter Erhalt einer ammoniakalischen Kupfersalzlösung gelöst. Der wässrige Ammoniak weist bevorzugt einen Massenanteil an Ammoniak im Bereich von 15 % bis 30 %, besonders bevorzugt im Bereich von 20 % bis 30 %, ganz besonders bevorzugt im Bereich von 25 % bis 30 %, auf.

Es empfiehlt sich, zur Stabilisierung des Kupfertetrammin-Komplexes einen pH-Wert (20°C) im Bereich von 7,0 bis 14, bevorzugt im Bereich von 8,0 bis 12, besonders bevorzugt im Bereich von 9,0 bis 11 einzuhalten. Es ist daher bevorzugt, einen Teil des zur Komplexierung benötigten Ammoniaks in Form von Ammoniumcarbonat oder Ammoniumacetat, bevorzugt Ammoniumcarbonat, zuzugeben und damit die Erhöhung des pH-Wertes zu begrenzen (Pufferung). Vorzugsweise wird so viel Ammoniumcarbonat oder Ammoniumacetat zugegeben, dass nach Zugabe des Kupfersalzes und Komplexierung der gewünschte pH-Wert erreicht wird. Durch Zugabe von wässrigem Ammoniak nach dem Mischen kann der pH-Wert nachträglich noch erhöht werden. Die Herstellung der Lösung des Kupfertetramminsalzes sollte bei tiefen Temperaturen erfolgen, um ein Ausgasen von Ammoniak zu verhindern. Temperaturen im Bereich von 0,0 °C bis 10,0 °C, bevorzugt im Bereich von 1,0 °C bis 5,0 °C haben sich bewährt.

Besonders bevorzugt wird wie folgt vorgegangen:

Das Kupfersalz, Ammoniumcarbonat und 80 % der berechneten Ammoniaklösung werden zunächst bei 5,0 °C vermischt. Unter Rühren wird so lange weitere (auf 5,0 °C gekühlte) Ammoniaklösung zugegeben, bis ein pH-Wert von 9,2 (siehe auch Hartinger, Handbuch Abwasser und Recyclingtechnik; Bild 2.25; S. 85; 2017; Günter Dietrich) erreicht ist.

Herstellung des Katalysators aus dem Träger und der Lösung des Kupfertetramminsalzes

Die zunächst erforderliche Tränkung des Trägers kann grundsätzlich nach bekannten Methoden erfolgen. Möglich ist sowohl die Anwendung der oben beschriebenen *incipient wetness*-Methode als auch die Tränkung in überstehender Lösung. Bevorzugt kommt die *incipient wetness*-Methode zum Einsatz, und zwar insbesondere in einer Variante, bei welcher der Träger in einem Tränkschritt (d. h. bei mehreren Tränkschritten: *in jedem der Tränkschritte*) nur mit einer solchen Menge der Tränklösung behandelt wird, die geringfügig (z. B. 2 % bis 5 %) unterhalb der zuvor bestimmten maximalen Saugfähigkeit des Trägers liegt (siehe den Beispielteil für die Bestimmung der maximalen Saugfähigkeit). Eine Tränkung in mehreren Schritten ist auch möglich, wenn besonders hohe Kupfergehalte angestrebt sind. Bevorzugt werden jedoch nicht mehr als zwei Tränkschritte durchgeführt. An die Tränkung schließt sich eine Trocknung (insbesondere bei Temperaturen im Bereich von 80 °C bis 150 °C, bevorzugt im Bereich von 90 °C bis 130 °C, besonders

bevorzugt im Bereich von 100 °C bis 120 °C) an. Hierdurch wird eine Vorstufe des Hydrierkatalysators erhalten.

Aus dieser Vorstufe wird durch Kalzinieren der *Kupfertetrammin-basierte Tränkkatalysator auf einem Träger umfassend Siliciumdioxid-Formkörper oder Siliciumcarbid-Formkörper* erhalten. Das Kalzinieren erfolgt dabei insbesondere bei Temperaturen im Bereich von 300 °C bis 600 °C, bevorzugt im Bereich von 350°C bis 550°C, besonders bevorzugt im Bereich von 400°C bis 500°C. Die Kalzinierung kann in einer Sauerstoff-haltigen Atmosphäre (insbesondere in Luft), in einer Wasserstoffatmosphäre oder in einer inerten Atmosphäre (insbesondere in Stickstoff- oder Edelgasatmosphäre) durchgeführt werden. Kalzinierung in Sauerstoff-haltiger Atmosphäre ist bevorzugt. Die Kalzinierungsbedingungen bestimmen, in welcher Form das Kupfer auf dem Katalysator vorliegt; bei Kalzinierung unter oxidierenden Bedingungen liegt überwiegend bis vollständig oxidisches Kupfer, insbesondere CuO, vor, bei Kalzinierung unter reduzierenden Bedingungen liegt überwiegend bis vollständig metallisches Kupfer vor.

Die Anzahl der Tränkungsschritte und die Konzentration der Lösung des Kupfertetramminsalzes werden bevorzugt so aufeinander abgestimmt, dass der Hydrierkatalysator, bezogen auf seine Gesamtmasse, nach dem Kalzinieren einen Massenanteil an (in dem Hydrierkatalysator insgesamt enthaltenen) Kupferverbindungen, berechnet als metallisches Cu, im Bereich von 3 % bis 35 %, bevorzugt 7 % bis 30 %, besonders bevorzugt 11 % bis 25 %, aufweist.

DURCHFÜHRUNG DER HYDRIERUNG

Nitroaromaten

Das erfindungsgemäße Verfahren ist grundsätzlich zur Hydrierung sämtlicher technisch relevanter Nitroaromaten zu den entsprechenden aromatischen Aminen geeignet. Besonders bevorzugt werden Nitroaromaten der folgenden Formel hydriert:

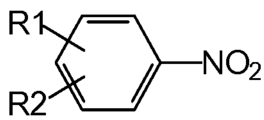

Dabei bedeuten R1 und R2 unabhängig voneinander Wasserstoff, Methyl oder Ethyl, wobei R2 weiterhin auch $NO_2$ bedeuten kann. Besonders bevorzugt ist die Hydrierung von Nitrobenzol (R1 = R2 =H) zu Anilin.

Aktivierung des Katalysators (optional als separater Schritt)

Vor Beginn der eigentlichen Hydrierung ist es bevorzugt, den Hydrierkatalysator mit Wasserstoff, insbesondere bei Temperaturen im Bereich von 180 °C bis 240 °C, zu reduzieren und so in seine "aktive Form" zu bringen *(zu aktivieren;* **Schritt (II)** des erfindungsgemäßen Verfahrens). Da der Katalysator auch bei der eigentlichen Hydrierung reduzierenden Bedingungen ausgesetzt ist, ist eine Aktivierung als ein der eigentlich Hydrierung vorgelagerter separater Schritt nicht unbedingt erforderlich. Die Durchführung des vorgelagerten Schrittes (II) führt jedoch zu verbesserten momentanen Selektivitäten zu Beginn der Hydrierung. Die Aktivierung erfolgt vorzugsweise nach Inertisierung des Reaktors durch ein Inertgas (insbesondere Stickstoff) mit einem Wasserstoffstrom eines Drucks im Bereich von 1,0 $bar_{(abs\cdot)}$ bis 3,0 $bar_{(abs\cdot)}$, beispielsweise 1,5 $bar_{(abs\cdot)}$, wobei durch schrittweise Zugabe des Wasserstoffs die Temperaturerhöhung auf dem Katalysator durch Aktivierung 30 °C nicht übersteigen sollte. Die Aktivierung erfolgt so lange, bis nach Zugabe von 100 % Wasserstoff keine exotherme Reaktion mehr zu beobachten ist. Vorzugsweise erfolgt dieser Schritt bereits in dem Reaktor, der für die eigentliche Hydrierung vorgesehen ist (siehe unten).

Hydrierung

**Schritt (III)** des erfindungsgemäßen Verfahrens, die Umsetzung der aromatischen Nitroverbindung mit Wasserstoff (= Hydrierung) in Gegenwart des, bevorzugt aktivierten, Hydrierkatalysators unter Erhalt des aromatischen Amins, kann grundsätzlich erfolgen wie aus dem Stand der Technik bekannt.

Die Hydrierung erfolgt in einem dafür vorgesehenen Apparat, dem Hydrierreaktor oder kurz Reaktor. Geeignete Reaktoren sind dem Fachmann hinlänglich bekannt.

Die Hydrierung der aromatischen Nitroverbindung erfolgt bevorzugt kontinuierlich. Wird der Wasserstoff überstöchiometrisch eingesetzt, so erfolgt bevorzugt eine Rückführung nicht umgesetzten Wasserstoffs in die Reaktion. Die Reaktion kann in der Flüssig- und in der Gasphase erfolgen. Die Durchführung der Reaktion in der Gasphase ist bevorzugt.

Die Hydrierung kann adiabatisch oder isotherm durchgeführt werden. Bei adiabatischer Betriebsweise erfolgt keine gezielte Wärmezufuhr oder Wärmeabfuhr. Daher spiegelt sich die Reaktionsenthalpie - abgesehen von unvermeidbaren Wärmeverlusten, zu deren Minimierung der Reaktor bevorzugt isoliert ist - quantitativ in der Temperaturdifferenz zwischen dem Edukt- und dem Produktgemisch wider (sog. *adiabatischer Temperatursprung*). Demgegenüber wird bei der isothermen Betriebsweise die Temperatur - abgesehen von unvermeidbaren lokalen sog. "Hotspots" - durch externe indirekte Kühlung konstant gehalten. Mögliche Reaktionsführungen, die auch im erfindungsgemäßen Verfahren angewandt werden können, sind in EP 0 944 578 A2 (isotherme Fahrweise) und in EP 0 696 574 A1, EP 0 696 573 A1, sowie in EP 1 882 681 A1 (adiabatische Fahrweise) beschrieben. Besonders bevorzugt werden folgende Bedingungen, die sich insbesondere für eine Reaktion in der Gasphase bewährt haben, eingehalten: Die Durchführung von Schritt (III) erfolgt

adiabatisch bei Temperaturen im Bereich von 160 °C bis 500 °C, bevorzugt 180 °C bis 450 °C, besonders bevorzugt 200 °C bis 400 °C,
oder

isotherm bei Temperaturen im Bereich von 180 °C bis 550 °C bevorzugt 200 °C bis 500 °C, besonders bevorzugt 220 °C bis 450 °C.

Bezüglich des molaren Verhältnisses von Wasserstoff zu Nitrogruppen versteht sich von selbst, dass dieses zur Erzielung vollständigen Umsatzes an Nitroaromaten mindestens stöchiometrisch, also mindestens 3 (= 3 : 1), sein muss. Es hat sich jedoch bewährt, Wasserstoff überstöchiometrisch einzusetzen, wobei der über die Stöchiometrie hinausgehende Überschuss bei adiabatischer Fahrweise im Allgemeinen besonders hoch gewählt wird, um die beträchtliche Reaktionswärme von diesem Überschusswasserstoff aufnehmen zu lassen. Insbesondere betrifft die vorliegende Erfindung daher auch ein Verfahren, bei welchem Schritt (III)

adiabatisch bei einem molaren Verhältnis von Wasserstoff zu Nitrogruppen im Bereich von 10 bis 200, bevorzugt von 20 bis 150, besonders bevorzugt von 60 bis 120,
oder

isotherm bei einem molaren Verhältnis von Wasserstoff zu Nitrogruppen im Bereich von 3 bis 100, bevorzugt von 6 bis 60, besonders bevorzugt von 10 bis 30,

durchgeführt wird.

Bevorzugte Reaktoren für einen isotherm betriebenen Reaktor sind thermostatisierte Rohr- oder Rohrbündelreaktoren. Geeignete Ausführungsformen solcher Reaktoren sind zum Beispiel in DE 2 201 528 A1, DE 2 207 166 A1, DE 198 06 810 A1, EP 1 439 901 A1, EP 1 569 745 A1, EP 1 590 076 A1, EP 1 587 612 A1, EP 1 586 370 A1, EP 1 627 678 A1 oder DE 202 006 014 116 U1 beschrieben.

Bevorzugte Reaktoren für einen adiabatisch betriebenen Reaktor sind die in DE 10 2006 035 203, Absätze [0030] bis [0033] beschriebenen.

Unabhängig von der Betriebsweise (isotherm oder adiabatisch) ist es bevorzugt, den Hydrierkatalysator in Schritt (III) in einem *Katalysatorfestbett* anzuordnen. Dies bedeutet, dass die Formkörper im eingesetzten Reaktor ortsfest vorliegen, zum Beispiel im Inneren thermostatisierter Reaktionsrohre (isotherm betriebene Rohr- und Rohbündelreaktoren) oder auf einem Tragerost, insbesondere zwischen zwei Tragerosten (adiabatisch betriebene Reaktoren enthaltend *Katalysatorschüttungen*) angeordnet sind. Das Gegenstück zu derartigen *Festbettreaktoren* sind *Wirbelschichtreaktoren* (wie sie zum Beispiel in WO 2010/130604 A2 zum Einsatz kommen), in denen sehr feinteilige Katalysatorpartikel (mit mittleren Größen im Mikrometerbereich) in wirbelnde Bewegung versetzt werden.

Nachfolgend wird die Erfindung mit Hilfe von Beispielen näher erläutert.

## Beispiele:

### Allgemeine Methoden

*Bestimmung der maximalen Saugfähigkeit des Trägers*

Die Bestimmung des Saugfähigkeitsmaximums erfolgt dabei durch Auswägen der Formkörper vor und nach Wasseraufnahme, wie nachfolgend beschrieben. Hierzu wird das Trägermaterial eingewogen und in einem visuelle Beobachtung ermöglichenden Gefäß (z. B. Becherglas) so lange mit vollentsalztem Wasser überschichtet stehen gelassen (ohne das Gefäß zu bewegen), bis keine Luftblasen mehr aufsteigen. Das überstehende Wasser wird dekantiert und die Oberfläche der noch feuchten Formkörper getrocknet. Dies geschieht durch Aufnehmen der oberflächlich anhaftenden Feuchtigkeit mit Filterpapier, was je nach Gestalt der Formkörper durch Rollen auf dem oder Abtupfen mit

dem Filterpapier durchgeführt werden kann. Durch diesen Trocknungsschritt wird *oberflächlich anhaftendes* Wasser entfernt, nicht jedoch Wasser, das in die Poren des Trägers aufgenommen wurde. Durch Auswägen des Inhaltes und Subtraktion der Einwaage erhält man die Wasseraufnahme in Gramm, was dem Saugfähigkeitsmaximum des eingesetzten Formkörpers entspricht.

In allen Beispielen zur Katalysatorpräparation wurde die Menge der zur Tränkung einzusetzenden Metallsalzlösung so abgestimmt, dass sie 2 % unterhalb der maximalen Saugfähigkeit lag *(incipient wetness-Methode).*

## Einsatzstoffe

$Cu(NO_3)_2$-Lösung der Firma *Poletto Aldo* mit einer Dichte bei 20 °C von 1,48 g/ml, einem Cu-Gehalt von (14,5 ± 0,5) Massen-% und einem pH-Wert (gemessen bei Umgebungstemperatur, 20 bis 25 °C) von 3,5 ± 0,5.

Siliciumdioxid-Formkörper, 3 × 5 mm-Zylinder, Saugfähigkeit 1,13 ml/g, Schüttgewicht 417 g/l.

## Beispiel 1: Herstellung eines Kupfernitrat-basierten Tränkkatalysators als Vergleichskatalysator

100 ml des Siliciumdioxid-Trägers wurden mit der $Cu(NO_3)_2$-Lösung getränkt. Dabei wurde die Mischung so lange bewegt, bis die Flüssigkeit vollständig vom Trägermaterial aufgenommen wurde. Die getränkten Formkörper wurden bei 120 °C bis zur Massenkonstanz getrocknet und anschließend für 4 h bei 450 °C in Luftatmosphäre kalziniert. Der Katalysator wies einen Massenanteil an Kupferverbindungen, berechnet als metallisches Kupfer, von ca. 24,0 % auf.

## Beispiel 2: Hydrierung von Nitrobenzol mit dem Katalysator aus Beispiel 1 (Vergleich)

Der Katalysator aus Beispiel 1 wurde im oxidierten Zustand in einen Festbettreaktor überführt und so lange mit Stickstoff durchströmt, bis der verbleibende Sauerstoff ausgetrieben war. Die Temperatur wurde auf einen Wert im Bereich von 200 °C bis 240 °C eingestellt und die Aktivierung gestartet, indem Wasserstoff zudosiert wurde. Die durch die Reaktion hervorgerufene Exothermie sollte so gering wie möglich gehalten werden. Nach Abschluss der Aktivierung wurde der Katalysator zur Entfernung des überschüssigen Wasserstoffs mit Stickstoff durchströmt. Für die Reaktion wurde Nitrobenzol (NB) zu dem aktivierten Katalysator dosiert, wobei die Nitrobenzolmenge sukzessive auf die Zielbelastung von 0,9 $g_{NB}$ $ml_{Kat}^{-1}$ $h^{-1}$ erhöht und angepasst wurde. Das molare Wasserstoff: Nitrobenzol-Verhältnis betrug 10:1. Die Reaktion wurde polytrop geführt, wobei ein Wärmeträger die bei der Reaktion entstandene Wärme abführte. Die Hydrierung wurde jeweils durchgeführt, bis Nitrobenzoldurchbruch beobachtet wurde.

Der in Beispiel 1 beschriebene Katalysator zeigte eine **Laufzeit von 60 h und eine durchschnittliche Anilinselektivität von 99,2 %.**

## Beispiel 3: Herstellung eines Kupfertetrammin-basierten Tränkkatalysators als Hydrierkatalysator für das erfindungsgemäße Verfahren

Eingesetzte Massen für eine Lösung mit einem Massenanteil an Cu von (12,7 ± 0,5) % bei pH = 9,2 ±1,0:

- Ammoniumcarbonat 65,785 g
- Basisches Kupfercarbonat 74,6 g
- Ammoniak 81,7 g
- VE-Wasser 100 g

Zuerst wurden die Einsatzstoffe im Kühlschrank auf unter 5 °C gekühlt. Wasser und Ammoniak wurden in einem verschließbaren Behälter vermischt. Die Feststoffe wurden in einer Schale gemeinsam eingewogen, zügig zu der gekühlten Ammoniaklösung gegeben und bei geschlossenem Deckel bis zur Auflösung der Salze vermischt.

100 ml des Silicumdioxid-Trägers wurden mit einer der Saugfähigkeit des Trägers entsprechenden Menge der so hergestellten Kupfertetrammincarbonat-Lösung getränkt. Dabei wurde die Mischung so lange bewegt, bis die Flüssigkeit vollständig vom Trägermaterial aufgenommen wurde. Die getränkten Formkörper wurden bei 120 °C bis zur Massenkonstanz getrocknet und anschließend für 4 h bei 450 °C kalziniert. Der Katalysator wies einen Massenanteil an Kupferverbindungen, berechnet als metallisches Kupfer, von ca. 14,8 % auf.

## Beispiel 4: Hydrierung von Nitrobenzol mit dem Katalysator aus Beispiel 3 (erfindungsgemäß)

Mit Ausnahme des Katalysators wurde der Versuch analog zu Beispiel 2 durchgeführt, wobei die **Laufzeit 295 h und die durchschnittliche Anilinselektivität 99,6%** betrug.

**Beispiel 5: Herstellung eines Kupfertetrammin-basierten Tränkkatalysators als Hydrierkatalysator für das erfindungsgemäße Verfahren bei pH 10**

Ausgehend von Beispiel 3 wurde ein Katalysator präpariert, der bei pH = 10 präpariert wurde. Folgende Mengen wurden verwendet:

- Ammoniumcarbonat: 15,8 g
- Basisches Kupfercarbonat: 18,18 g
- Ammoniak: 32,60 g
- VE-Wasser: 33,42 g

Zuerst wurden die Einsatzstoffe im Kühlschrank auf unter 5 °C gekühlt. Wasser und Ammoniak werden in einem verschließbaren Behälter vermischt. Die Feststoffe wurden in einer Schale gemeinsam eingewogen, zügig zu der gekühlten Ammoniaklösung gegeben und bei geschlossenem Deckel bis zur Auflösung der Salze vermischt.

100 ml des Silicumdioxid-Trägers wurden mit einer der Saugfähigkeit des Trägers entsprechenden Menge der so hergestellten Kupfertetrammincarbonat-Lösung getränkt. Dabei wurde die Mischung so lange bewegt, bis die Flüssigkeit vollständig vom Trägermaterial aufgenommen wurde. Die getränkten Formkörper wurden bei 120 °C bis zur Massenkonstanz getrocknet und anschließend für 4 h bei 450 °C kalziniert. Der Katalysator wies einen Massenanteil an Cu von ca. 12,4 % auf.

**Beispiel 6: Hydrierung von Nitrobenzol mit dem Katalysator aus Beispiel 5 (erfindungsgemäß)**

Mit Ausnahme des Katalysators wurde der Versuch analog zu Beispiel 2 durchgeführt, wobei die die **Laufzeit 240 h betrug und die durchschnittliche Anilinselektivität 99,6%.**

**Beispiel 7: Herstellung eines Kupfertetrammin-basierten Tränkkatalysators als Hydrierkatalysator für das erfindungsgemäße Verfahren bei pH 10 inkl. "Mauken"**

Es wurde ein Katalysator analog zu Beispiel 5 hergestellt, mit dem Unterschied, dass der Katalysator im Tränkprozess vor der Trocknung eine Woche im nassen Zustand stehen gelassen wurde.

**Beispiel 8: Hydrierung von Nitrobenzol mit dem Katalysator aus Beispiel 7 (erfindungsgemäß)**

Mit Ausnahme des Katalysators wurde der Versuch analog zu Beispiel 2 durchgeführt, wobei die **Laufzeit 240 h betrug und die durchschnittliche Anilinselektivität einen Wert von 99,5% aufwies.**

**Beispiel 9: Herstellung eines Kupfertetrammin-basierten Tränkkatalysators als Hydrierkatalysator für das erfindungsgemäße Verfahren bei pH 10 mit Mehrfachimprägnierung**

Es wurde analog zu Beispiel 5 verfahren, jedoch wurde durch zweifache Imprägnierung ein höherer Kupfergehalt erzielt. Der Massenanteil an Kupferverbindungen, berechnet als metallisches Kupfer, nach Kalzinierung betrug ca. 22 %.

**Beispiel 10: Hydrierung von Nitrobenzol mit dem Katalysator aus Beispiel 9 (erfindungsgemäß)**

Mit Ausnahme des Katalysators wurde der Versuch analog zu Beispiel 2 durchgeführt, wobei die **Laufzeit 360 h betrug und die durchschnittliche Anilinselektivität 99,6%.**

**Beispiel 11: Herstellung eines Kupfertetrammin-basierten Tränkkatalysators als Hydrierkatalysator für das erfindungsgemäße Verfahren bei pH 9,2 mit Mehrfachimprägnierung bei geringerer Metallkonzentration**

Es wurde analog zu Beispiel 5 verfahren, jedoch wurde eine zweifache Imprägnierung bei geringerem Metallgehalt der Imprägnierlösung durchgeführt. Der Massenanteil an Kupferverbindungen, berechnet als metallisches Kupfer, nach Kalzinierung betrug ca. 15,3 %.

**Beispiel 12: Hydrierung von Nitrobenzol mit dem Katalysator aus Beispiel 11 (erfindungsgemäß)**

Mit Ausnahme des Katalysators wurde der Versuch analog zu Beispiel 2 durchgeführt, wobei die **Laufzeit 290**

**h betrug und die durchschnittliche Anilinselektivität einen Wert von 99,6% aufwies.**

**Beispiel 13: Herstellung eines Kupfertetrammin-basierten Tränkkatalysators als Hydrierkatalysator für das erfindungsgemäße Verfahren bei pH 9,2 auf einem alternativen Silicaträger**

Es wurde analog zu Beispiel 3 verfahren, jedoch wurde ein alternatives Silicaträgermaterial mit geringerer spezifischer Oberfläche von 80 $m^2/g$ eingesetzt. Der Massenanteil an Kupferverbindungen, berechnet als metallisches Kupfer, nach Kalzinierung betrug ca. 12,9 %.

**Beispiel 14: Hydrierung von Nitrobenzol mit dem Katalysator aus Beispiel 13 (erfindungsgemäß)**

Mit Ausnahme des Katalysators wurde der Versuch analog zu Beispiel 2 durchgeführt, wobei die **Laufzeit 260 h betrug und die durchschnittliche Anilinselektivität einen Wert von 99,5% aufwies.**

**Beispiel 15: Herstellung eines Kupfertetrammin-basierten Tränkkatalysators** als **Hydrierkatalysator für das erfindungsgemäße Verfahren bei pH 10 auf einem "Trilobe"-Formkörper**

Es wurde analog zu Beispiel 5 verfahren, jedoch wurde als Trägermaterial ein Trilobe-Formkörper auf Silicabasis eingesetzt. Der Massenanteil an Kupferverbindungen, berechnet als metallisches Kupfer, nach Kalzinierung betrug ca. 11,6 %.

**Beispiel 16: Hydrierung von Nitrobenzol mit dem Katalysator aus Beispiel 15 (erfindungsgemäß)**

Mit Ausnahme des Katalysators wurde der Versuch analog zu Beispiel 2 durchgeführt, wobei die **Laufzeit 240 h betrug und die durchschnittliche Anilinselektivität 99,7%.**

**Beispiel 17: Herstellung eines Kupfertetrammin-basierten Tränkkatalysators als Hydrierkatalysator für das erfindungsgemäße Verfahren bei pH 10 mit Mehrfachtränkung auf einem Trilobe- Formkörper**

Es wurde analog zu Beispiel 5 verfahren, jedoch wurde als Trägermaterial ein Trilobe-Formkörper auf Silicabasis eingesetzt und eine Mehrfachtränkung durchgeführt. Der Massenanteil an Kupferverbindungen, berechnet als metallisches Kupfer, nach Kalzinierung betrug ca. 19,6 %.

**Beispiel 18: Hydrierung von Nitrobenzol mit dem Katalysator aus Beispiel 17 (erfindungsgemäß)**

Mit Ausnahme des Katalysators wurde der Versuch analog zu Beispiel 2 durchgeführt, wobei die **Laufzeit 300 h betrug und die durchschnittliche Anilinselektivität einen Wert von 99,7% aufwies.**

**Beispiel 19: Herstellung eines Kupfertetrammin-basierten Tränkkatalysators als Hydrierkatalysator für das erfindungsgemäße Verfahren bei pH 10 mit Mehrfachtränkung auf einem Siliciumcarbidträger von vergleichsweise niedriger Reinheit (ca. 99,5 % SiC)**

Es wurde analog zu Beispiel 5 verfahren, jedoch wurde als Trägermaterial ein SiliciumcarbidTräger (ca. 99,5 % SiC) eingesetzt und eine Mehrfachtränkung durchgeführt. Der Massenanteil an Kupferverbindungen, berechnet als metallisches Kupfer, nach Kalzinierung betrug ca. 10,3 %.

**Beispiel 20: Hydrierung von Nitrobenzol mit dem Katalysator aus Beispiel 19 (erfindungsgemäß)**

Mit Ausnahme des Katalysators wurde der Versuch analog zu Beispiel 2 durchgeführt, wobei die **Laufzeit 290 h betrug und die durchschnittliche Anilinselektivität einen Wert von 99,8% aufwies.**

**Beispiel 21: Herstellung eines Kupfertetrammin-basierten Tränkkatalysators als Hydrierkatalysator für das erfindungsgemäße Verfahren bei pH 10 mit Mehrfachtränkung auf einem Siliciumcarbidträger von im Vergleich zu Beispiel 19 höherer Reinheit (> 99,85 % SiC)**

Es wurde analog zu Beispiel 5 verfahren, jedoch wurde als Trägermaterial ein SiliciumcarbidTräger (≥ 99,85 % SiC) eingesetzt und eine Mehrfachtränkung durchgeführt. Der Massenanteil an Kupferverbindungen, berechnet als metallisches Kupfer, nach Kalzinierung betrug ca. 10,1 %.

**Beispiel 22: Hydrierung von Nitrobenzol mit dem Katalysator aus Beispiel 21 (erfindungsgemäß)**

Mit Ausnahme des Katalysators wurde der Versuch analog zu Beispiel 2 durchgeführt, wobei die **Laufzeit 240 h betrug und die durchschnittliche Anilinselektivität einen Wert von 99,8% aufwies.**

## Patentansprüche

1. Verfahren zur Herstellung eines aromatischen Amins durch Hydrierung einer aromatischen Nitroverbindung, umfassend die Schritte:

    (I) Bereitstellen eines Kupfertetramminsalz-basierten Tränkkatalysators umfassend ein Metall oder Metalloxid auf einem Träger als Hydrierkatalysator, wobei mindestens metallisches oder oxidisches Kupfer zugegen ist und der auf alle vorhandenen Metalle bezogene Stoffmengenanteil von Cu im Bereich von 0,75 bis 1 liegt, und wobei der Träger Siliciumdioxid-Formkörper oder Siliciumcarbid-Formkörper umfasst;
    (II) optional, Aktivieren des Hydrierkatalysators durch Behandeln mit Wasserstoff in Abwesenheit der aromatischen Nitroverbindung;
    (III) Umsetzen der aromatischen Nitroverbindung mit Wasserstoff in Gegenwart des, gegebenenfalls aktivierten, Hydrierkatalysators unter Erhalt des aromatischen Amins.

2. Verfahren gemäß Anspruch 1, bei welchem Schritt (II) durchgeführt wird und die Behandlung mit Wasserstoff bei Temperaturen im Bereich von 180 °C bis 240 °C erfolgt.

3. Verfahren gemäß einem der vorstehenden Ansprüche, bei welchem Schritt (III)

    adiabatisch bei Temperaturen im Bereich von 160 °C bis 500 °C,
    oder
    isotherm bei Temperaturen im Bereich von 180 °C bis 550 °C,

    durchgeführt wird.

4. Verfahren gemäß einem der vorstehenden Ansprüche, bei welchem Schritt (III)

    adiabatisch bei einem molaren Verhältnis von Wasserstoff zu Nitrogruppen im Bereich von 10 bis 200,
    oder
    isotherm bei einem molaren Verhältnis von Wasserstoff zu Nitrogruppen im Bereich von 3 bis 100,

    durchgeführt wird.

5. Verfahren gemäß einem der vorstehenden Ansprüche, bei welchem der Massenanteil an Kupferverbindungen, berechnet als metallisches Cu, des in (I) bereitgestellten Hydrierkatalysators, bezogen auf dessen Gesamtmasse, im Bereich von 3 % bis 35 % liegt.

6. Verfahren gemäß einem der vorstehenden Ansprüche, bei welchem als Hydrierkatalysator ein Kupfertetrammincarbonat-basierter Tränkkatalysator eingesetzt wird.

7. Verfahren gemäß Anspruch 6, bei welchem als Hydrierkatalysator ein Kupfertetrammincarbonat-Ammoniumcarbonat-basierter Tränkkatalysator oder ein Kupfertetrammincarbonat-Ammoniumacetat-basierter Tränkkatalysator eingesetzt wird.

8. Verfahren nach einem der vorstehenden Ansprüche, bei welchem der Tränkkatalysator erhältlich ist durch ein Verfahren, in welchem der Träger so mit einer wässrigen Lösung eines Kupfertetramminsalzes getränkt wird, dass die mittels Sättigung mit Wasser bestimmte maximale Saugfähigkeit des Trägers nicht überschritten wird.

9. Verfahren gemäß einem der vorstehenden Ansprüche, bei welchem Schritt (I) umfasst:

    (a) Lösen eines Kupfersalzes in wässrigem Ammoniak unter Erhalt einer ammoniakalischen Kupfersalzlösung;
    (b) Tränken des Trägers mit der in (a) erhaltenen ammoniakalischen Kupfersalzlösung gefolgt von Trocknen

des so erhaltenen getränkten Trägers unter Erhalt einer Katalysatorvorstufe,
(c) Kalzinieren der in (c) erhaltenen Katalysatorvorstufe unter Ausbildung des Kupfertetrammin-basierten Tränkkatalysators.

**10.** Verfahren gemäß Anspruch 9, bei welchem die zum Tränken eingesetzte ammoniakalische Kupfersalzlösung einen pH-Wert (20 °C) im Bereich von 7,0 bis 14 aufweist.

**11.** Verfahren gemäß einem der vorstehenden Ansprüche, bei welchem eine aromatische Nitroverbindung der Formel

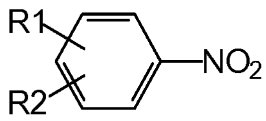

hydriert wird, worin R1 und R2 unabhängig voneinander Wasserstoff, Methyl oder Ethyl bedeuten, wobei R2 weiterhin auch $NO_2$ bedeuten kann.

**12.** Verfahren nach einem der Ansprüche 9 bis 11, bei welchem der Träger in Schritt (b) so mit der in (a) erhaltenen ammoniakalischen Kupfersalzlösung getränkt wird, dass die mittels Sättigung mit Wasser bestimmte maximale Saugfähigkeit des Trägers nicht überschritten wird.

**13.** Verfahren nach Anspruch 12, bei welchem die maximale Saugfähigkeit des Trägers um nicht mehr als 5 % unterschritten wird.

**14.** Verfahren nach Anspruch 12 oder 13, bei welchem die maximale Saugfähigkeit um mindestens 2 % unterschritten wird.

**15.** Verfahren gemäß einem der vorstehenden Ansprüche, bei welchem der, gegebenenfalls aktivierte, Hydrierkatalysator in Schritt (III) in einem Katalysatorfestbett angeordnet wird.

**Claims**

**1.** Process for preparing an aromatic amine by hydrogenating an aromatic nitro compound, comprising the steps of

(I) providing a tetraamminecopper salt-based impregnated catalyst comprising a metal or metal oxide on a support as hydrogenation catalyst, where at least metallic or oxidic copper is present and the molar proportion of Cu based on all metals present is in the range from 0.75 to 1, and where the carrier comprises shaped silicon dioxide bodies or shaped silicon carbide bodies;
(II) optionally activating the hydrogenation catalyst by treating with hydrogen in the absence of the aromatic nitro compound;
(III) reacting the aromatic nitro compound with hydrogen in the presence of the optionally activated hydrogenation catalyst to obtain the aromatic amine.

**2.** Process according to Claim 1, in which step (II) is conducted and the treatment with hydrogen is effected at temperatures in the range from 180°C to 240°C.

**3.** Process according to either of the preceding claims, in which step (III) is conducted

adiabatically at temperatures in the range from 160°C to 500°C,
or
isothermally at temperatures in the range from 180°C to 550°C.

**4.** Process according to any of the preceding claims, in which step (III) is conducted

adiabatically at a molar ratio of hydrogen to nitro groups in the range from 10 to 200,
or
isothermally at a molar ratio of hydrogen to nitro groups in the range from 3 to 100.

5. Process according to any of the preceding claims, in which the proportion by mass of copper compounds, calculated as metallic Cu, in the hydrogenation catalyst provided in (I), based on the total mass thereof, is in the range from 3% to 35%.

6. Process according to any of the preceding claims, in which the hydrogenation catalyst used is a tetraamminecopper carbonate-based impregnated catalyst.

7. Process according to Claim 6, in which the hydrogenation catalyst used is a tetraamminecopper carbonate/ammonium carbonate-based impregnated catalyst or a tetraamminecopper carbonate/ammonium acetate-based impregnated catalyst.

8. Process according to any of the preceding claims, in which the impregnated catalyst is obtainable by a process in which the support is impregnated with an aqueous solution of a tetraamminecopper salt, in such a way as not to exceed the maximum absorptivity of the support determined by means of saturation with water.

9. Process according to any of the preceding claims, in which step (I) comprises:

(a) dissolving a copper salt in aqueous ammonia to obtain an ammoniacal copper salt solution;
(b) impregnating the support with the ammoniacal copper salt solution obtained in (a), followed by drying of the impregnated support thus obtained to obtain a catalyst precursor,
(c) calcining the catalyst precursor obtained in (c) to form the tetraamminecopper-based impregnated catalyst.

10. Process according to Claim 9, in which the ammoniacal copper salt solution used for impregnation has a pH (20°C) in the range from 7.0 to 14.

11. Process according to any of the preceding claims, in which an aromatic nitro compound of the formula

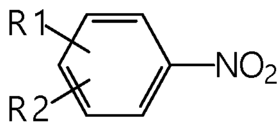

is hydrogenated, in which R1 and R2 are independently hydrogen, methyl or ethyl, where R2 may additionally also be $NO_2$.

12. Process according to any of Claims 9 to 11, in which the support in step (b) is impregnated with the ammoniacal copper salt solution obtained in (a) in such a way as not to exceed the maximum absorptivity of the support determined by means of saturation with water.

13. Process according to Claim 12, in which the maximum absorptivity of the support is undershot by not more than 5%.

14. Process according to Claim 12 or 13, in which the maximum absorptivity is undershot by at least 2%.

15. Process according to any of the preceding claims, in which the optionally activated hydrogenation catalyst in step (III) is disposed in a fixed catalyst bed.

## Revendications

1. Procédé de préparation d'une amine aromatique par hydrogénation d'un composé nitré aromatique, comprenant les étapes suivantes :

(I) fourniture, en tant que catalyseur d'hydrogénation, d'un catalyseur imprégné à base d'un sel de cuivre-tétrammine comprenant un métal ou un oxyde métallique sur un support, en présence d'au moins un cuivre métallique ou oxyde, et qui présente une proportion pondérale de Cu, rapportée à tous les métaux présents, dans la plage de 0,75 à 1, et le support comprenant un objet moulé en dioxyde de silicium ou un objet moulé en carbure de silicium ;
(II) éventuellement, activation du catalyseur d'hydrogénation par traitement à l'hydrogène en l'absence du

composé nitré aromatique ;
(III) réaction du composé nitré aromatique avec de l'oxygène en présence du catalyseur d'hydrogénation éventuellement activé, avec obtention de l'amine aromatique.

2. Procédé selon la revendication 1, dans lequel l'étape (II) est mise en oeuvre, et le traitement à l'hydrogène a lieu à des températures dans la plage de 180 °C à 240 °C.

3. Procédé selon l'une des revendications précédentes, dans lequel l'étape (III) est mise en oeuvre

dans des conditions adiabatiques à des températures dans la plage de 160 °C à 500 °C,
ou
dans des conditions isothermes à des températures dans la plage de 180 °C à 550 °C.

4. Procédé selon l'une des revendications précédentes, dans lequel l'étape (III) est mise en oeuvre

dans des conditions adiabatiques pour un rapport en moles de l'hydrogène aux groupes nitro dans la plage de 10 à 200,
ou
dans des conditions isothermes pour un rapport en moles de l'hydrogène aux groupes nitro dans la plage de 3 à 100.

5. Procédé selon l'une des revendications précédentes, dans lequel la proportion en masse des composés du cuivre, exprimée en Cu métallique, du catalyseur d'hydrogénation fourni en (I), par rapport à la masse totale, est comprise dans la plage de 3 % à 35 %.

6. Procédé selon l'une des revendications précédentes, dans lequel on utilise comme catalyseur d'hydrogénation un catalyseur imprégné à base de carbonate de cuivre-tétrammine.

7. Procédé selon la revendication 6, dans lequel on utilise comme catalyseur d'hydrogénation un catalyseur imprégné à base de carbonate de cuivre-tétrammine-carbonate d'ammonium ou un catalyseur imprégné à base de carbonate de cuivre-tétrammine-acétate d'ammonium.

8. Procédé selon l'une des revendications précédentes, dans lequel le catalyseur imprégné peut être obtenu par un procédé dans lequel le support est imprégné d'une solution aqueuse d'un sel de cuivre-amine de façon à ne pas dépasser la capacité d'absorption maximale, déterminée par saturation à l'eau, du support.

9. Procédé selon l'une des revendications précédentes, dans lequel l'étape (I) comprend :

(a) la dissolution d'un sel de cuivre dans de l'ammoniaque avec obtention d'une solution ammoniacale d'un sel de cuivre ;
(b) imprégnation du support avec la solution ammoniacale de cuivre obtenue en (a), suivie du séchage du support imprégné ainsi obtenu, avec obtention d'un précurseur de catalyseur,
(c) calcination du précurseur de catalyseur obtenu en (c), avec formation du catalyseur imprégné à base de cuivre-tétrammine.

10. Procédé selon la revendication 9, dans lequel la solution ammoniacale d'un sel de cuivre utilisée pour l'imprégnation présente un pH (20 °C) dans la plage de 7,0 à 14.

11. Procédé selon l'une des revendications précédentes, dans lequel on hydrogène un composé nitré aromatique de formule

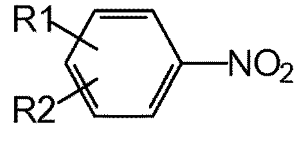

dans laquelle R1 et R2 représentent indépendamment l'un de l'autre un hydrogène, un méthyle ou un éthyle, R2

pouvant en outre représenter aussi $NO_2$.

12. Procédé selon l'une des revendications 9 à 11, dans lequel le support est imprégné dans l'étape (b) de la solution ammoniacale de cuivre obtenue en (a) de façon à ne pas dépasser la capacité d'absorption maximale, déterminée par saturation à l'eau, du support.

13. Procédé selon la revendication 12, dans lequel la capacité d'absorption du support est de pas plus 5 % inférieure à la capacité maximale.

14. Procédé selon la revendication 12 ou 13, dans lequel la capacité d'absorption est d'au moins 2 % inférieure à la capacité maximale.

15. Procédé selon l'une des revendications précédentes, dans lequel le catalyseur d'hydrogénation, éventuellement activé, est dans l'étape (III) disposé dans un lit fixe de catalyseur.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2849002 A1
- DE 19715746 A1
- EP 1882681 A1
- WO 2013030221 A1
- US 1207802 A
- US 3136818 A
- GB 823026 A
- US 2891094 A
- WO 2010130604 A2
- EP 0696573 A1
- DE 2311114
- WO 9532171 A1
- WO 2009027135 A1
- WO 9853910 A1
- DE 3933661 A1
- DE 102010029924 A1
- GB 825602 A
- EP 3320969 A1
- US 3900504 A
- GB 961394 A
- EP 0944578 A2
- EP 0696574 A1
- DE 2201528 A1
- DE 2207166 A1
- DE 19806810 A1
- EP 1439901 A1
- EP 1569745 A1
- EP 1590076 A1
- EP 1587612 A1
- EP 1586370 A1
- EP 1627678 A1
- DE 202006014116 U1
- DE 102006035203


### In der Beschreibung aufgeführte Nicht-Patentliteratur


- *Chem. Lett.,* 1980, 1197-1200
- *Appl. Catal,* 1982, vol. 3, 381-388
- *Appl. Catal.,* 1987, vol. 31, 309-321
- *Procedia Engineering,* 2013, vol. 51, 467-472
- *Pure & Appl. Chem.,* 1994, vol. 66, 1739-1758
- **F. M. NELSEN ; F. T. EGGERTSEN.** *Analyt. Chem.,* 1958, vol. 30, 1387-1392
- **S. W. SING.** Adsorption, Surface Area and Porosity. 1982
- **HARTINGER.** Handbuch Abwasser und Recyclingtechnik. 2017, 85